# EUROPÄISCHE PATENTSCHRIFT

(11) **EP 3 415 970 B1**
(45) Veröffentlichungstag und Bekanntmachung des Hinweises auf die Patenterteilung: **18.03.2020**
(21) Anmeldenummer: 18176480.4
(22) Anmeldetag: 07.06.2018
(51) Int. Cl.: G02B 23/24, A61B 1/00, A61B 1/002, G02B 13/00, G02B 1/11

(54) **OBJEKTIV FÜR EIN ENDOSKOP UND ENDOSKOP**
LENS FOR AN ENDOSCOPE AND ENDOSCOPE
OBJECTIF POUR UN ENDOSCOPE ET ENDOSCOPE

(30) Priorität: 16.06.2017 DE 102017113273
(43) Veröffentlichungstag der Anmeldung: 19.12.2018
(73) Patentinhaber: avateramedical GmbH, 07745 Jena (DE)
(72) Erfinder: Khettal, Ali, 12435 Berlin (DE); Weise, Fabian, 10437 Berlin (DE)
(74) Vertreter: Schaumburg und Partner Patentanwälte mbB

(56) Entgegenhaltungen:
- JP-A- H05 297 272
- US-A- 4 621 910

## Beschreibung

Die Erfindung betrifft ein Objektiv für ein Endoskop, mit einem objektseitigen Linsenglied, das eine plan-konvexe Stablinse enthält, und einem bildseitigen Linsenglied, das eine am bildseitigen Ende des Objektivs angeordnete bikonvexe Linse enthält. Ferner betrifft die Erfindung ein monokulares Endoskop oder stereoskopisches Endoskop.

Endoskope werden insbesondere in der minimalinvasiven Chirurgie verwendet, um dem operierenden Arzt Einblick in die Körperregion zu gewähren, in der das Operationsgebiet liegt. Am distalen Ende eines Endoskopschafts ist typischerweise ein Objektiv angeordnet, welches das vom zu beobachtenden Objekt ausgehende Licht sammelt und ein reelles Zwischenbild des Objektes erzeugt. Dieses Zwischenbild wird mit Hilfe eines dem Objektiv nachgeordneten optischen Relaissystems zum proximalen Ende des Endoskopschafts übertragen. Am proximalen Ende des Endoskopschafts ist ein Okular angeordnet, welches das reelle Zwischenbild entweder für das menschliche Auge oder mittels eines Kameraobjektivs auf eine Sensoroberfläche abbildet.

Insbesondere bei starren Endoskopen ist es oft schwierig oder gar unmöglich, das Endoskop so auszurichten, dass das zu beobachtende Objekt auf der Achse des starren Endoskopschafts liegt. Typischerweise werden hierzu Objektive gefertigt, welche mindestens eine Strahlumlenkung vorsehen und somit eine Beobachtung von Objekten ermöglichen, die nicht auf der Achse des Endoskopschafts liegen.

Endoskope, bei denen die optische Achse objektseitiger Linsenelemente gegenüber der Achse des Endoskopschafts abgewinkelt ist, werden im Folgenden auch als Schrägsicht-Endoskope bezeichnet. Im Gegensatz dazu werden Endoskope, die nur eine Beobachtung von Objekten erlauben, die im Wesentlichen auf der Achse des Endoskopschafts liegen, als Geradsicht-Endoskope bezeichnet.

Aus dem Dokument CN 105093515 A ist ein Objektiv für ein Geradsicht-Endoskop bekannt, welches zwei Linsenglieder umfasst, von denen das objektseitig angeordnete Linsenglied einen Glasstab sowie eine plan-konvexe Stablinse und das bildseitig angeordnete Linsenglied eine bikonvexe Linse enthält. Das Dokument CN 105093515 A offenbart ferner ein Objektiv für ein Schrägsicht-Endoskop. Die dazu notwendige Strahlumlenkung wird mithilfe eines Prismas realisiert.

In dem Dokument US 5,051,824 A wird ebenfalls ein Objektiv für ein Schrägsicht-Endoskop offenbart.

Dokument US 4,621,910 offenbart ein mehrgliedriges Objektiv für ein Endoskop.

Ausgehend von dem bekannten Stand der Technik ist es Aufgabe der Erfindung, ein Objektiv für ein Endoskop anzugeben, das einen einfachen und kompakten Aufbau bei hoher optischer Qualität besitzt.

Diese Aufgabe wird durch ein Objektiv mit den Merkmalen des Anspruchs 1 und ein monokulares Endoskop oder stereoskopisches Endoskop mit den Merkmalen des Anspruchs 15 gelöst. Vorteilhafte Weiterbildungen sind in den abhängigen Ansprüchen angegeben.

Das erfindungsgemäße Objektiv für ein Endoskop umfasst ein objektseitiges Linsenglied, das eine plan-konvexe Stablinse enthält, und ein bildseitiges Linsenglied, das eine am bildseitigen Ende des Objektivs angeordnete bikonvexe Linse enthält. Das objektseitige Linsenglied enthält eine plan-konvexe erste Linse und eine bikonkave zweite Linse, die eine Frontlinse bilden und in dieser Reihenfolge objektseitig der plan-konvexen Stablinse, die eine dritte Linse bildet, angeordnet sind. Das bildseitige Linsenglied enthält von der Objektseite her gesehen eine bikonvexe vierte Linse, eine konkav-plane fünfte Linse und eine bikonkave sechste Linse, die in dieser Reihenfolge objektseitig der am bildseitigen Ende des Objektivs angeordneten bikonvexen Linse, die eine siebente Linse bildet, angeordnet sind.

Die optischen Elemente des erfindungsgemäßen Objektivs wirken vorteilhaft zusammen, um ein Zwischenbild hoher optischer Qualität zu erzeugen. Insbesondere erzeugt die spezielle Ausgestaltung des bildseitigen Linsenglieds ein Zwischenbild mit einer definierten, negativen Bildfeldwölbung. Diese Bildfeldwölbung kann durch weitere optische Elemente, insbesondere ein optisches Relaissystem und ein Okular, derart korrigiert werden, dass eine Abbildung ohne signifikante Bildfeldwölbung (bzw. mit einer geringen Verzeichnung und/oder frei von Astigmatismus) erhalten wird. Das erfindungsgemäße Objektiv umfasst ferner nur wenige Bauelemente bzw. optische Bauteile und hat dadurch einen einfachen und kompakten Aufbau. Hierdurch wird ein einfacher und kompakter Aufbau bei hoher optischer Qualität erreicht. Insbesondere hat das erfindungsgemäße Objektiv einen vergleichsweise geringen Durchmesser, beispielsweise bei einem Sichtfeld (FOV) größer als 70°.

In einer vorteilhaften Weiterbildung sind die bikonvexe vierte Linse, die konkav-plane fünfte Linse, die bikonkave sechste Linse und/oder die bikonvexe siebente Linse des bildseitigen Linsenglieds miteinander verkittet. Durch die Verwendung verkitteter Linsen, im Folgenden auch als Kittglieder bezeichnet, lässt sich der Herstellungs- und Montageaufwand erheblich verringern. Insbesondere kann das gesamte bildseitige Linsenglied als ein einziges Bauelement ausgebildet sein.

In einer weiteren vorteilhaften Ausgestaltung sind die plan-konvexe erste Linse und die bikonkave zweite Linse des objektseitigen Linsenglieds miteinander verkittet. Diese beiden Linsen zusammen werden im Folgenden auch als Frontlinse bezeichnet. Die Frontlinse kann insbesondere als Achromat ausgebildet sein. Ferner kann die bikonkave zweite Linse mit der plan-konvexen dritten Linse verkittet sein. Dadurch kann das objektseitige Linsenglied als ein einziges Bauelement ausgebildet sein.

Es ist vorteilhaft, wenn die optische Achse der Frontlinse in Bezug auf eine Längsachse eines Endoskopschafts des Endoskop abgewinkelt ist, wenn zwischen der bikonkaven zweiten Linse und der plan-konvexen dritten Linse ein Prisma derart angeordnet ist, dass es eine Strahlumlenkung von der optischen Achse der Frontlinse auf die Längsachse des Endoskopschafts des Endoskop bewirkt, und wenn das Prisma mit der plan-konvexen dritten Linse verkittet ist. Ausführungsbeispiele mit einem Prisma zur Strahlumlenkung werden im Folgenden auch als Schrägsicht-Objektive bezeichnet, um sie von Geradsicht-Objektiven ohne Prisma zu unterscheiden.

In einer vorteilhaften Weiterbildung des Schrägsicht-Objektivs ist zwischen der bikonkaven zweiten Linse und dem Prisma eine planparallele Glasplatte angeordnet, die mit der bikonkaven zweiten Linse und dem Prisma verkittet ist. Die planparallele Glasplatte kann insbesondere derart ausgebildet sein, dass die Länge des Glaswegs des Schrägsicht-Objektivs auf die Länge des Glaswegs des Geradsicht-Objektivs verlängert wird. Ist die Länge des Glaswegs des objektseitigen Linsenglieds in dem Geradsicht-Objektiv gleich der Länge des Glaswegs in dem Schrägsicht-Objektiv, so kann das bildseitige Linsenglied in beiden Ausführungsformen identisch ausgebildet sein.

In einer vorteilhaften Weiterbildung des Geradsicht-Objektivs ist zwischen der Frontlinse und der plan-konvexen dritten Linse ein Glasstab, insbesondere mit zwei planparallelen Flächen, angeordnet. Der Glasstab kann mit der plan-konvexen dritten Linse und der bikonkaven zweiten Linse verkittet sein. Durch die Anordnung des Glasstabs wird erreicht, dass die plan-konvexe dritte Linse sowohl in Ausführungsbeispielen zur Geradsicht als auch in Ausführungsbeispielen zur Schrägsicht dieselbe Länge besitzt, ohne dass die Länge des Objektivs insgesamt angepasst werden muss.

Vorzugsweise bestehen die plan-konvexe erste Linse, die bikonkave zweite Linse, der Glasstab und/oder die plan-konvexe dritte Linse des objektseitigen Linsenglieds und/oder das Prisma und/oder die planparallele Glasplatte und/oder die bikonvexe vierte Linse, die konkav-plane fünfte Linse, die bikonkave sechste Linse und/oder die bikonvexe siebente Linse des bildseitigen Linsenglieds aus einem Flintglas.

Die plan-konvexe erste Linse, der Glasstab, die plan-konvexe dritte Linse, die planparallele Glasplatte, die bikonvexe vierte Linse, die konkav-plane fünfte Linse, die bikonkave sechste Linse und/oder die bikonvexe siebente Linse weisen beispielsweise eine Antireflexbeschichtung auf. Die Antireflexbeschichtung dient der Verminderung von Streulicht und einer damit einhergehenden Verschlechterung der optischen Qualität des Objektivs. Ferner erhöht die Antireflexbeschichtung auch die optische Transmission.

Das Prisma weist insbesondere eine Hochreflexionsbeschichtung auf denjenigen Flächen auf, an denen Licht zur Strahlumlenkung reflektiert wird. Eine solche Beschichtung vermindert den Lichtverlust durch Transmission bei der Reflexion.

In einer vorteilhaften Weiterbildung sind die plan-konvexe erste Linse, die bikonkave zweite Linse, das Prisma und/oder die planparallele Glasplatte von einem Material derart umgeben, dass der jeweiliger Durchmesser des Elements auf den Durchmesser der plan-konvexen dritten Linse und/oder des Glasstabs angeglichen wird. Somit kann erreicht werden, dass die optischen Bauteile des Objektivs einen gemeinsamen Durchmesser besitzen, was die Montage erleichtert und die optische und mechanische Stabilität erhöht.

Ferner ist es vorteilhaft, wenn objektseitig der Frontlinse eine planparallele Scheibe angeordnet ist, welche beispielsweise aus Saphir besteht. Die Verwendung von Saphir ist aufgrund seiner optischen Eigenschaften, insbesondere des hohen Transmissionsgrads in den relevanten Wellenlängen, seiner chemischen Eigenschaften (insbesondere ist Saphir chemisch inert) und seiner mechanischen Eigenschaften, insbesondere seiner Kratz- und Abriebbeständigkeit, vorteilhaft.

In einer weiteren vorteilhaften Weiterbildung weist das Objektiv eine mechanische Trennstelle zwischen dem objektseitigen Linsenglied und dem bildseitigen Linsenglied auf. Die mechanische Trennstelle gestattet es, das objektseitige Linsenglied in besonders einfacher Weise durch ein anderweitig ausgebildetes objektseitiges Linsenglied zu ersetzen. Durch das Vorhalten von verschiedenen, mechanisch gefassten objektseitigen Linsengliedern in Ausgestaltungen für Geradsicht und Schrägsicht lassen sich besonders einfach Endoskope mit verschiedenen Sichtweisen realisieren.

Die Erfindung betrifft ferner ein stereoskopisches Objektiv zur Verwendung in einem stereoskopischen Endoskop vor. Das stereoskopische Objektiv umfasst zwei Objektive nach oben beschriebener Art.

Ein weiterer Aspekt der Erfindung betrifft ein monokulares Endoskop oder stereoskopisches Endoskop. Das monokulare Endoskop umfasst ein im Vorhergehenden beschriebenes Objektiv. Das stereoskopische Endoskop umfasst das gerade genannte stereoskopische Objektiv.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Figur 1: ein Ausführungsbeispiel eines Objektivs für ein Endoskop;
- Figur 2: ein Ausführungsbeispiel eines bildseitigen Linsenglieds für das Objektiv nach Figur 1; und
- Figur 3: ein Ausführungsbeispiel eines monokularen Endoskops, das ein Objektiv nach Figur 1 enthält; und
- Figur 4: ein Ausführungsbeispiel eines stereoskopischen Endoskops, das zwei Ob-jektive nach Figur 1 enthält.

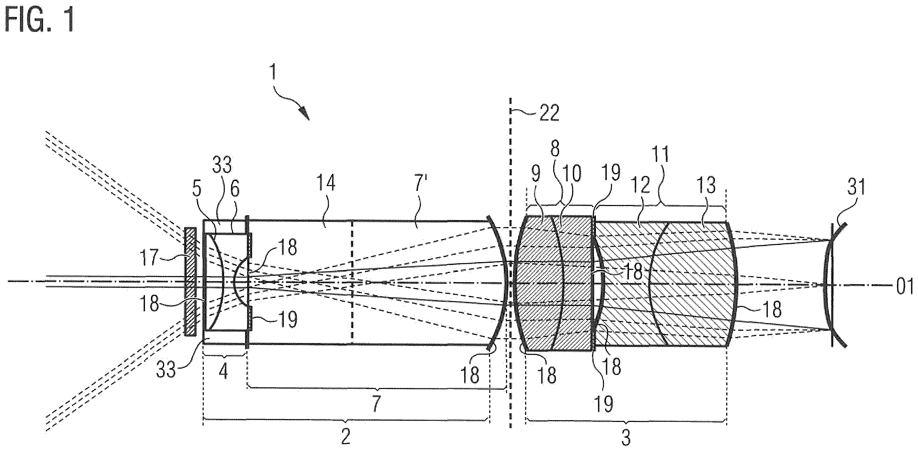

Figur 1 zeigt ein Ausführungsbeispiel eines Objektivs 1 für ein Endoskop 24, 25 in schematischer Darstellung. In dem gezeigten Ausführungsbeispiel ist das Objektiv 1 ein Geradsicht-Objektiv, d.h. das gezeigte Ausführungsbeispiel ist für die Beobachtung von Objekten geeignet, die im Wesentlichen auf einer Achse O1 eines in Figur 1 nicht gezeigten Endoskopschafts liegen.

Das Objektiv 1 enthält ein objektseitig angeordnetes Linsenglied 2 und ein bildseitig angeordnetes Linsenglied 3. Das objektseitige Linsenglied 2 ist von dem bildseitigen Linsenglied durch eine mechanische Trennstelle 22 getrennt.

Das objektseitige Linsenglied 2 umfasst von der Objektseite her gesehen eine Frontlinse 4, einen Glasstab 14 mit zwei planparallelen Flächen und eine plankonvexe dritte Linse 7, die als Stablinse ausgebildet ist. Die Frontlinse 4 ist durch eine plan-konvexe erste Linse 5 und eine bikonkave zweite Linse 6 gebildet. Die beiden Linsen 5, 6 der Frontlinse 4 sind miteinander verkittet. Die Frontlinse 4 ist mit dem Glasstab 14 verkittet. Ferner ist der Glasstab 14 mit der plan-konvexen dritten Linse 7 verkittet, so dass das objektseitige Linsenglied 2 ein einziges Bauteil bildet. Objektseitig der Frontlinse 4 ist ein Saphirglasfenster 17 angeordnet.

Das bildseitige Linsenglied 3 umfasst von der Objektseite her gesehen eine erste Linsengruppe 8 und eine zweite Linsengruppe 11. Die erste Linsengruppe 8 umfasst von der Objektseite her gesehen eine bikonvexe vierte Linse 9 und eine konkav-plane fünfte Linse 10, die miteinander verkittet sind. Die zweite Linsengruppe 11 umfasst von der Objektseite her gesehen eine bikonkave sechste Linse 12 und eine bikonvexe siebente Linse 13, welche ebenfalls miteinander verkittet sind. Die beiden Linsengruppen 8, 11 des bildseitigen Linsenglieds 3 sind beispielsweise durch Verkitten der konkav-planen fünften Linse 10 und der bikonkaven sechsten Linse 12 miteinander verbunden, so dass das bildseitige Linsenglied 3 ein einziges Bauelement bildet. Im Ausführungsbeispiel nach Figur 1 sind die beiden Linsengruppen 8, 11 des bildseitigen Linsenglieds 3 hingegen nicht verkittet.

Die Frontlinse 4, die plan-konvexe dritte Linse 7, die bikonvexe vierte Linse 9 der ersten Linsengruppe 8, die konkav-plane fünfte Linse 10 der ersten Linsengruppe 8 und die bikonvexe siebente Linse 13 der zweiten Linsengruppe 11 weisen an unverkitteten Oberflächen eine Antireflexionsbeschichtung 18 auf. Ferner weist das Objektiv 1 auf der bikonkaven zweiten Linse 6 und der konkav-planen fünften Linse 10 geschwärzte Flächen 19 auf.

Die erste Linsengruppe 8 und die zweite Linsengruppe 11 des bildseitigen Linsenglieds 3 bilden für sich genommen jeweils eine achromatische Feldlinse. Das bildseitige Linsenglied 3 erzeugt ein chromatisch korrigiertes Zwischenbild 31 mit einer definierten, negativen Bildfeldwölbung. Die mechanische Trennstelle 22 gestattet den einfachen Austausch des objektseitigen Linsenglieds 2. Die geschwärzten Flächen 19 wirken in dem Objektiv 1 als Blenden (insbesondere jeweils als Blende für die Streulichtminimierung und nicht als Feldblende oder Apertur).

Tabelle 1 zeigt die Linsendaten des Objektivs 1 nach Figur 1. Die optisch wirksamen Flächen sind in Tabelle 1 von der Objektseite her mit 1 bis 10 durchnummeriert. Alle Längenangaben sind in der Einheit [mm]. Die Bezeichnung der Gläser folgt der Nomenklatur von Schott.

In Tabelle 2 sind die paraxialen optischen Daten des Objektivs 1 nach Figur 1 gezeigt.

**Tabelle 1**

| Fläche | Radius | Dicke | Glas | Durchmesser |
|---|---|---|---|---|
| Objekt | Unendlich | 50 | | 74,43 |
| 1 | Unendlich | 0,5 | N-LASF46A | 2,8 |
| 2 | -2,84 | 0,3 | N-BAF4 | 2,8 |
| 3 | 8,85 | 0,368 | | 1,4 |
| 4 | Unendlich | 7,3 | N-LASF44 | 3,6 |
| 5 | -3,15 | 0,306 | | 3,6 |
| 6 | 5,4 | 1,4 | N-LAF21 | 3,6 |
| 7 | -3,7 | 0,7 | N-SF6 | 3,6 |
| 8 | Unendlich | 0,447 | | 3,6 |
| 9 | -2,92 | 1,1 | N-SF1 | 3,6 |
| 10 | 2,92 | 2,5 | N-LASF31 | 3,6 |

**Tabelle 2**

| | |
|---|---|
| Brennweite | 1,91 mm |
| NA | 0,086 |
| Feldwinkel | 72 |
| ø Bild (diagonal) | 2,50 mm |
| Radius der Bildfeldwölbung | -2,65 mm |
| ø Optik | 3,60 mm |

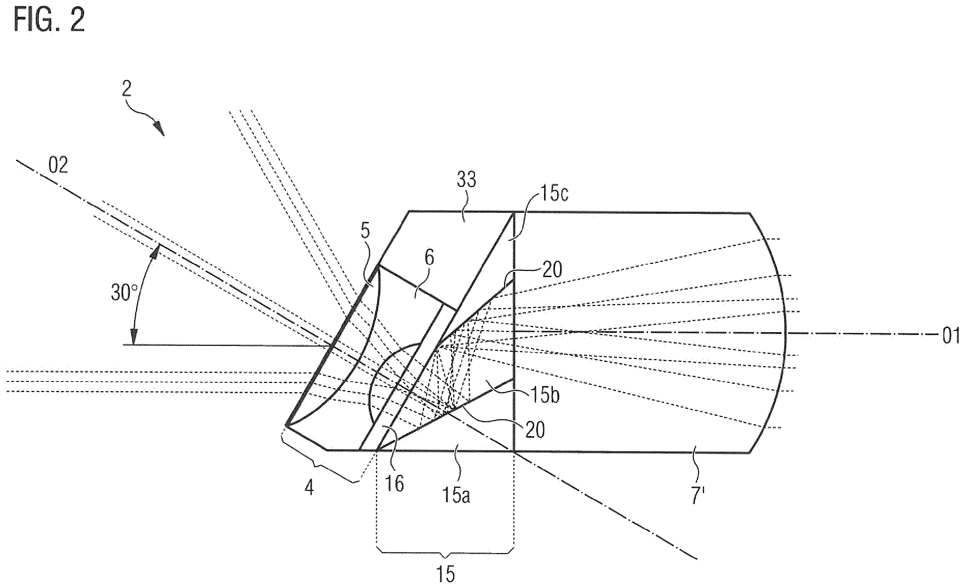

Figur 2 zeigt ein Ausführungsbeispiel des objektseitigen Linsenglieds 2 für das Objektiv 1 nach Figur 1. Das in Figur 2 gezeigte, objektseitige Linsenglied 2 ist für die Verwendung in einem Schrägsicht-Objektiv geeignet. Das objektseitige Linsenglied 2 nach Figur 2 unterscheidet sich von dem objektseitigen Linsenglied 2 nach Figur 1 im Wesentlich durch ein zwischen der Frontlinse 4 und der plan-konvexen dritten Linse 7 angeordnetes Prisma 15. Ferner umfasst das objektseitige Linsenglied 2 hier eine planparallele Glasplatte 16, die zwischen der Frontlinse 4 und dem Prisma 15 angeordnet ist. Das Prisma 15 ist durch drei Elemente 15a, 15b, 15c gebildet, von denen nur eins (15b) optisch wirksam ist. Die Flächen des Prismas 15, die in das Objektiv 1 einfallendes Licht reflektieren, weisen beispielsweise eine Hochreflexbeschichtung 20 auf. In dem gezeigten Ausführungsbeispiel haben die zwei außenliegenden Elemente 15a, 15c hingegen einen niedrigeren Brechungsindex als das innenliegende Element 15b, wodurch eine Totalreflexion an den Grenzflächen realisiert werden kann. Eine Hochreflexbeschichtung ist daher im Ausführungsbeispiel nach Figur 1 nicht unbedingt erforderlich.

Das Prisma 15 realisiert eine Strahlumlenkung von der optischen Achse O2 der Frontlinse 4 auf die Achse O1 des in Figur 2 nicht gezeigten Endoskopschafts. Die Verkippung erlaubt eine Beobachtung von Objekten, die nicht auf der Achse O1 des Endoskopschafts liegen. Gezeigt ist beispielhaft eine Verkippung der Achsen um 30°, es sind aber auch andere Verkippungswinkel denkbar, beispielsweise 15°, 45° oder 90°. Die planparallele Glasplatte 16 sorgt dafür, dass die Länge des Glaswegs des objektseitigen Linsenglieds 2 nach Figur 2 der Länge des Glaswegs des objektseitigen Linsenglieds 2 nach Figur 1 entspricht. Dabei entspricht der Glasweg insbesondere dem Weg, den das Licht innerhalb der optischen Elemente zurücklegt. Dadurch kann in beiden Ausführungsbeispielen das in Figur 1 gezeigte bildseitige Linsenglied 3 zusammen mit dem jeweiligen objektseitigen Linsenglied 2 verwendet werden. Die mechanische Trennstelle 22 des Objektivs 1 nach Figur 1 gestattet den Austausch des objektseitigen Linsenglieds 2 des Objektivs 1, beispielsweise durch das objektseitige Linsenglied 2 nach Figur 2, um ein Endoskop 24 mit Schrägsicht zu realisieren. Das Endoskop 24 kann somit einfach an unterschiedliche Bedürfnisse angepasst werden.

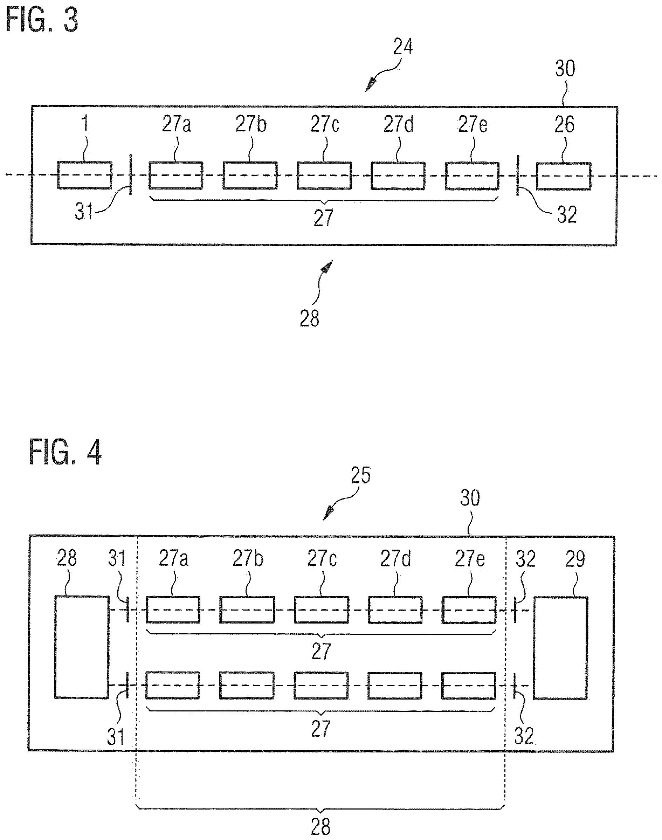

In Figur 3 ist ein Ausführungsbeispiel eines monokularen Endoskops 24 gezeigt, welches das Objektiv 1 nach Figur 1 enthält. Das monokulare Endoskop 24 umfasst von der Objektseite her gesehen ein Objektiv 1, ein optisches Relaissystem 28 mit einem Relaismodul 27 mit mehreren Relaismodulkomponenten 27a bis 27e und ein Okular 26. Ferner weist das Endoskop 24 einen Schaft 30 auf, in dem die vorgenannten Elemente angeordnet sind.

Das am distalen Ende des Endoskops 24 angeordnete Objektiv 1 erzeugt ein erstes Zwischenbild 31 des zu beobachtenden Objektes. Das Relaissystem 27 bildet das distale, erste Zwischenbild 31 auf ein proximales, zweites Zwischenbild 32 ab. Damit überträgt das Relaissystem 27 das erste Zwischenbild 31 gleichsam vom distalen zum proximalen Ende des Endoskops 24. Das am proximalen Ende des Endoskops 24 angeordnete Okular 26 bildet schließlich das zweite Zwischenbild 32 auf einen in Figur 3 nicht gezeigten Kamerasensor ab.

Das von dem Objektiv 1 erzeugte distale Zwischenbild 31 weist eine negative Bildfeldwölbung auf. Das optische Relaissystem 28 ist ausgestaltet, die negative Bildfeldwölbung des Objektivs 1 zu korrigieren. Die Abbildung des Endoskops 24 weist somit keine oder nur eine vernachlässigbare Bildfeldwölbung bei kompaktem Aufbau aller optischen Bauteile auf.

Ein Ausführungsbeispiel eines stereoskopischen Endoskops 25 ist in Figur 4 schematisch dargestellt. Gegenüber dem in Figur 3 dargestellten monokularen Endoskop 24 weist das stereoskopische Endoskop 25 zwei optische Kanäle auf. Das stereoskopische Endoskop 25 hat einen Schaft 30, in dem vom distalen Ende her betrachtet ein Objektiv 23, ein optisches Relaissystem 28 mit zwei Relaismodulen 27 für jeden der beiden optischen Kanäle (stereoskopisches Relaissystem) und ein proximal angeordnetes Okular 29 angeordnet sind.

Das Objektiv 23 ist durch zwei Objektive 1 nach Figur 1 gebildet. Jeweils eines der beiden Objektive 1 ist dabei einem der optischen Kanäle zugeordnet. Jedes der beiden Objektive 1 erzeugt aus dem zu beobachtenden Objekt ein erstes Zwischenbild 31. Das stereoskopische Relaissystem 28 nach Figur 2 bildet je eines der beiden distalen Zwischenbilder 31 auf jeweils eines der beiden proximalen Zwischenbilder 32 ab. Die dadurch erzeugten proximalen Zwischenbilder 32 werden dann durch das Okular 29 auf einen in Figur 4 nicht gezeigten Kamerasensor abgebildet.

### Bezugszeichenliste

- 1: Objektiv
- 2: objektseitige Linsenglied
- 3: bildseitige Linsenglied
- 4: Frontlinse
- 5: plan-konvexe erste Linse
- 6: bikonkave zweite Linse
- 7, 7': plan-konvexe dritte Linse (Stablinse)
- 8: erste Linsengruppe
- 9: bikonvexe vierte Linse
- 10: konkav-plane fünfte Linse
- 11: zweite Linsengruppe
- 12: bikonkave sechste Linse
- 13: bikonvexe siebente Linse
- 14: Glasstab
- 15: Prisma
- 16: planparallele Glasplatte
- 17: Saphirglasfenster
- 18: Antireflexbeschichtung
- 19: geschwärzte Fläche
- 20: Hochreflexbeschichtung
- 22: mechanische Trennstelle
- 23: stereoskopisches Objektiv
- 24: monokulares Endoskop
- 25: stereoskopisches Endoskop
- 26, 29: Okular
- 27: Relaismodul
- 28: Relaissystem
- 30: Schaft
- 31, 32: Zwischenbild
- O1: Achse des Endoskopschafts
- O2: optische Achse der Frontlinse

## Patentansprüche

1. Objektiv (1) für ein Endoskop (24, 25), mit einem objektseitigen Linsenglied (2), das eine plan-konvexe Stablinse (7) enthält, und einem bildseitigen Linsenglied (3), das eine am bildseitigen Ende des Objektivs (1) angeordnete bikonvexe Linse (13) enthält, **dadurch gekennzeichnet, dass** das objektseitige Linsenglied (2) eine plan-konvexe erste Linse (5) und eine bikonkave zweite Linse (6) enthält, die eine Frontlinse (4) bilden und in dieser Reihenfolge objektseitig der plan-konvexen Stablinse (7), die eine dritte Linse bildet, angeordnet sind, und dass das bildseitige Linsenglied (3) von der Objektseite her gesehen eine bikonvexe vierte Linse (9), eine konkav-plane fünfte Linse (10) und eine bikonkave sechste Linse (12) enthält, die in dieser Reihenfolge objektseitig der am bildseitigen Ende des Objektivs (1) angeordneten bikonvexen Linse (13), die eine siebente Linse bildet, angeordnet sind.

2. Objektiv (1) nach Anspruch 1, **dadurch gekennzeichnet, dass** die plan-konvexe erste Linse (5) und die bikonkave zweite Linse (6) und die plankonvexe dritte Linse (7) des objektseitigen Linsenglieds (2) miteinander verkittet sind.

3. Objektiv (1) nach Anspruch 1 oder 2, **dadurch gekennzeichnet, dass** die bikonvexe vierte Linse (9), die konkav-plane fünfte Linse (10), die bikonkave sechste Linse (12) und/oder die bikonvexe siebente Linse (13) des bildseitigen Linsenglieds (3) miteinander verkittet sind.

4. Objektiv (1) nach einem der Ansprüche 1 bis 3, **dadurch gekennzeichnet, dass** die plankonvexe dritte Linse (7) durch eine plankonvexe Stablinse (7') und einen Glasstab (14), die miteinander verkittet sind, gebildet ist.

5. Objektiv (1) nach Anspruch 4, **dadurch gekennzeichnet, dass** der Glasstab (14) aus einem Flintglas besteht und/oder eine Antireflexionsbeschichtung (18) aufweist.

6. Objektiv (1) nach einem der Ansprüche 1 bis 3, **dadurch gekennzeichnet, dass** die optische Achse (O2) der Frontlinse (4) in Bezug auf eine Längsachse (O1) eines Endoskopschafts des Endoskop (24, 25) abgewinkelt ist, dass zwischen der bikonkaven zweiten Linse (6) und der plan-konvexen dritten Linse (7) ein Prisma (15) derart angeordnet ist, dass es eine Strahlumlenkung von der optischen Achse (O2) der Frontlinse (4) auf die Längsachse (O1) des Endoskopschafts des Endoskop (24, 25) bewirkt, und dass das Prisma (15) mit der plan-konvexen dritten Linse (7) verkittet ist.

7. Objektiv (1) nach Anspruch 6, **dadurch gekennzeichnet, dass** zwischen der bikonkaven zweiten Linse (6) und dem Prisma (15) eine planparallele Glasplatte (16) angeordnet und mit diesen verkittet ist.

8. Objektiv (1) nach Anspruch 6 oder 7, **dadurch gekennzeichnet, dass** das Prisma (15) eine Hochreflexionsbeschichtung (20) aufweist.

9. Objektiv (1) nach einem der Ansprüche 1 bis 8, **dadurch gekennzeichnet, dass** die plan-konvexe erste Linse (5), die bikonkave zweite Linse (6), die plan-konvexe dritte Linse (7), die bikonvexe vierte Linse (9), die konkav-plane fünfte Linse (10), die bikonkave sechste Linse (12) und/oder die bikonvexe siebente Linse (13) aus Flintglas bestehen.

10. Objektiv (1) nach einem der Ansprüche 1 bis 9, **dadurch gekennzeichnet, dass** die plan-konvexe erste Linse (5), die bikonkave zweite Linse (6), die plan-konvexe dritte Linse (7), die bikonvexe vierte Linse (9), die konkav-plane fünfte Linse (10), die bikonkave sechste Linse (12) und/oder die bikonvexe siebente Linse (13) eine Antireflexionsbeschichtung (18) aufweisen.

11. Objektiv (1) nach einem der Ansprüche 1 bis 10, **dadurch gekennzeichnet, dass** die plan-konvexe erste Linse (5) und die bikonkave zweite Linse (6) von einem Material (33) derart radial umgeben sind, dass der Durchmesser der Frontlinse (4) und der Durchmesser der plan-konvexen dritten Linse (7) angeglichen werden.

12. Objektiv (1) nach einem der Ansprüche 1 bis 11, **dadurch gekennzeichnet, dass** objektseitig der plan-konvexen ersten Linse (5) ein Saphirglasfenster (17) angeordnet ist.

13. Objektiv (1) nach einem der Ansprüche 1 bis 12, **dadurch gekennzeichnet, dass** zwischen dem objektseitigen Linsenglied (2) und dem bildseitigen Linsenglied (3) eine mechanische Trennstelle (22) angeordnet ist, die dazu ausgebildet ist, das objektseitige Linsenglied (2) auszutauschen.

14. Stereoskopisches Objektiv (23), umfassend zwei Objektive (1) nach einem der Ansprüche 1 bis 13.

15. Monokulares Endoskop (24) oder stereoskopisches Endoskop (25), **dadurch gekennzeichnet, dass** das monokulare Endoskop (24) ein Objektiv (1) nach einem der Ansprüche 1 bis 13 umfasst, und dass das stereoskopische Endoskop (25) das stereoskopisches Objektiv (23) nach Anspruch 14 umfasst.

## Claims

1. An objective lens (1) for an endoscope (24, 25) comprises an object-side lens element (2) including a plane-convex rod lens (7) and an image-side lens element (3) including a biconvex lens (13) arranged at the image-side end of the objective lens (1), **characterized in that** the object-side lens element (2) includes a plane-convex first lens (5) and a biconcave second lens (6) which form a front lens (4) and are arranged in this order on the object side of the plane-convex rod lens (7) forming a third lens, and that as viewed from the object side, the image-side lens element (3) includes a biconvex fourth lens (9), a concave-plane fifth lens (10) and a biconcave sixth lens (12) which are arranged in this order on the object side of the biconvex lens (13) that is arranged at the image-side end of the objective lens (1) and forms a seventh lens.

2. The objective lens (1) according to claim 1, **characterized in that** the plane-convex first lens (5) and the biconcave second lens (6) and the plane-convex third lens (7) of the object-side lens element (2) are cemented to each other.

3. The objective lens (1) according to claim 1 or 2, **characterized in that** the biconvex fourth lens (9), the concave-plane fifth lens (10), the biconcave sixth lens (12) and/or the biconvex seventh lens (13) of the image-side lens element (3) are cemented to each other.

4. The objective lens (1) according to one of the claims 1 to 3, **characterized in that** the plane-convex third lens (7) is formed by a plane-convex rod lens (7') and a glass rod (14) which are cemented to each other.

5. The objective lens (1) according to claim 4, **characterized in that** the glass rod (14) is made of flint glass and/or has an antireflection coating (18).

6. The objective lens (1) according to one of the claims 1 to 3, **characterized in that** the optical axis (O2) of the front lens (4) is angled with respect to a longitudinal axis (01) of an endoscope shaft of the endoscope (24, 25), that between the biconcave second lens (6) and the plane-convex third lens (7) a prism (15) is arranged such that it causes a beam deflection from the optical axis (O2) of the front lens (4) to the longitudinal axis (O1) of the endoscope shaft of the endoscope (24, 25), and that the prism (15) is cemented to the plane-convex third lens (7).

7. The objective lens (1) according to claim 6, **characterized in that** between the biconcave second lens (6) and the prism (15) a plane-parallel glass plate (16) is arranged and cemented thereto.

8. The objective lens (1) according to claim 6 or 7, **characterized in that** the prism (15) has a high-reflection coating (20).

9. The objective lens (1) according to one of the claims 1 to 8, **characterized in that** the plane-convex first lens (5), the biconcave second lens (6), the plane-convex third lens (7), the biconvex fourth lens (9), the concave-plane fifth lens (10), the biconcave sixth lens (12) and/or the biconvex seventh lens (13) are made of flint glass.

10. The objective lens (1) according to one of the claims 1 to 9, **characterized in that** the plane-convex first lens (5), the biconcave second lens (6), the plane-convex third lens (7), the biconvex fourth lens (9), the concave-plane fifth lens (10), the biconcave sixth lens (12) and/or the biconvex seventh lens (13) have an antireflection coating (18).

11. The objective lens (1) according to one of the claims 1 to 10, **characterized in that** the plane-convex first lens (5) and the biconcave second lens (6) are radially surrounded by a material (33) such that the diameter of the front lens (4) and the diameter of the plane-convex third lens (7) are matched.

12. The objective lens (1) according to one of the claims 1 to 11, **characterized in that** a sapphire glass window (17) is arranged on the object side of the plane-convex first lens (5).

13. The objective lens (1) according to one of the claims 1 to 12, **characterized in that** between the object-side lens element (2) and the image-side lens element (3) a mechanical separation point (22) is arranged which is formed to exchange the object-side lens element (2).

14. A stereoscopic objective lens (23), comprising two objective lenses (1) according to one of the claims 1 to 13.

15. A monocular endoscope (24) or stereoscopic endoscope (25), **characterized in that** the monocular endoscope (24) comprises an objective lens (1) according to one of the claims 1 to 13, and that the stereoscopic endoscope (25) comprises the stereoscopic objective lens (23) according to claim 14.

## Revendications

1. Objectif (1) pour un endoscope (24, 25), avec un organe formant lentille côté objet (2), qui contient une lentille en barre plan-convexe (7), et un organe formant lentille côté image (3), qui contient une lentille biconvexe (13) disposée à l'extrémité côté image de l'objectif (1), **caractérisé en ce que** l'organe formant lentille côté objet (2) contient une première lentille plan-convexe (5) et une deuxième lentille biconcave (6), qui forment une lentille frontale (4) et sont disposées dans cet ordre côté objet de la lentille en barre plan-convexe (7), qui forme une troisième lentille, et que l'organe formant lentille côté image (3) contient, vu à partir du côté objet, une quatrième lentille biconvexe (9), une cinquième lentille plan-concave (10) et une sixième lentille biconcave (12), qui sont disposées dans cet ordre côté objet de la lentille biconvexe (13), disposée à l'extrémité côté image de l'objectif (1), qui forme une septième lentille.

2. Objectif (1) selon la revendication 1, **caractérisé en ce que** la première lentille plan-convexe (5), la deuxième lentille biconcave (6) et la troisième lentille plan-convexe (7) de l'organe formant lentille côté objet (2) sont solidarisées les unes avec les autres.

3. Objectif (1) selon la revendication 1 ou 2, **caractérisé en ce que** la quatrième lentille biconvexe (9), la cinquième lentille plan-concave (10), la sixième lentille biconcave (12) et/ou la septième lentille biconvexe (13) de l'organe formant lentille côté image (3) sont solidarisées les unes avec les autres.

4. Objectif (1) selon l'une quelconque des revendications 1 à 3, **caractérisé en ce que** la troisième lentille plan-convexe (7) est formée par une lentille en barre plan-convexe (7') et une barre en verre (14), qui sont solidarisées les unes avec les autres.

5. Objectif (1) selon la revendication 4, **caractérisé en ce que** la barre en verre (14) est constituée d'un verre Flint et/ou présente un revêtement antireflet (18).

6. Objectif (1) selon l'une quelconque des revendications 1 à 3, **caractérisé en ce que** l'axe optique (O2) de la lentille frontale (4) est coudé par rapport à un axe longitudinal (O1) d'une tige d'endoscope de l'endoscope (24, 25), qu'un prisme (15) est disposé entre la deuxième lentille biconcave (6) et la troisième lentille plan-convexe (7), de telle sorte qu'une déviation de faisceau depuis l'axe optique (O2) de la lentille frontale (4) sur l'axe longitudinal (O1) de la tige d'endoscope de l'endoscope (24, 25) est provoquée, et que le prisme (15) est solidarisé avec la troisième lentille plan-convexe (7).

7. Objectif (1) selon la revendication 6, **caractérisé en ce qu'**une plaque de verre (16) à flans parallèles est disposée entre la deuxième lentille biconcave (6) et le prisme (15) et est solidarisée avec ceux-ci.

8. Objectif (1) selon la revendication 6 ou 7, **caractérisé en ce que** le prisme (15) présente un revêtement hautement réfléchissant (20).

9. Objectif (1) selon l'une quelconque des revendications 1 à 8, **caractérisé en ce que** la première lentille plan-convexe (5), la deuxième lentille biconcave (6), la troisième lentille plan-convexe (7), la quatrième lentille biconvexe (9), la cinquième lentille plan-concave (10), la sixième lentille biconcave (12) et/ou la septième lentille biconvexe (13) sont constituées de verre Flint.

10. Objectif (1) selon l'une quelconque des revendications 1 à 9, **caractérisé en ce que** la première lentille plan-convexe (5), la deuxième lentille biconcave (6), la troisième lentille plan-convexe (7), la quatrième lentille biconvexe (9), la cinquième lentille plan-concave (10), la sixième lentille biconcave (12) et/ou la septième lentille biconvexe (13) présentent un revêtement antireflet (18).

11. Objectif (1) selon l'une quelconque des revendications 1 à 10, **caractérisé en ce que** la première lentille plan-convexe (5) et la deuxième lentille biconcave (6) sont entourées radialement par un matériau (33), de telle sorte que le diamètre de la lentille frontale (4) et le diamètre de la troisième lentille plan-convexe (7) sont adaptés.

12. Objectif (1) selon l'une quelconque des revendications 1 à 11, **caractérisé en ce qu'**une fenêtre en verre de saphir (17) est disposée côté objet de la première lentille plan-convexe (5).

13. Objectif (1) selon l'une quelconque des revendications 1 à 12, **caractérisé en ce qu'**un point de séparation mécanique (22), qui est conçu pour remplacer l'organe formant lentille côté objet (2), est disposé entre l'organe formant lentille côté objet (2) et l'organe formant lentille côté image (3).

14. Objectif stéréoscopique (23), comprenant deux objectifs (1) selon l'une quelconque des revendications 1 à 13.

15. Endoscope monoculaire (24) ou endoscope stéréoscopique (25), **caractérisé en ce que** l'endoscope monoculaire (24) comprend un objectif (1) selon l'une quelconque des revendications 1 à 13, et que l'endoscope stéréoscopique (25) comprend l'objectif stéréoscopique (23) selon la revendication 14.
